# EUROPEAN PATENT SPECIFICATION

(11) **EP 3 090 741 B1**
(45) Date of publication and mention of the grant of the patent: **10.07.2019**
(21) Application number: 16425039.1
(22) Date of filing: 03.05.2016
(51) Int. Cl.: A61K 31/203, A61K 45/06, A61P 31/10

(54) **THE DIRECT FUNGISTATIC EFFECT OF ALL-TRANSRETINOIC ACID ON CANDIDA ALBICANS AND ASPERGILLUS FUMIGATUS**
DIREKTE FUNGISTATISCHE WIRKUNG VON RETINSÄURE AUF CANDIDA ALBICANS UND ASPERGILLUS FUMIGATUS
EFFET FONGISTATIQUE DIRECT D'ACIDE TOUT TRANS-RÉTINOÏQUE SUR CANDIDA ALBICANS ET ASPERGILLUS FUMIGATUS

(30) Priority: 05.05.2015 IT RM20150054
(43) Date of publication of application: 09.11.2016
(73) Proprietor: Orlandi, Augusto, 00184 Roma (IT); Campione, Elena, 00142 Roma (IT)
(72) Inventor: CAMPIONE, Elena, 00142 Roma (IT); ORLANDI Augusto, 00184 Roma (IT); GAZIANO, Roberta, 00049 Roma (IT); MARINO, Daniele, 00167 Roma (IT)
(74) Representative: Zwicker, Jörk

(56) References cited:
- WO-A1-97/02030
- WO-A1-03/039418
- WO-A1-03/094969
- HANDOJO I ET AL: "THE EFFECT OF TOPICAL RETINOIC ACID (AIROL) IN THE TREATMENT OF TINEA VERSICOLOR", SOUTHEAST ASIAN JOURNAL OF TROPICAL MEDICINE AND PUBLIC HEALTH, MINISTERS OF EDUCATION ORGANISATION * REGIONAL TROPICAL MEDICINE & PUBLIC HEALTH PROJECT, TH, vol. 8, no. 1, 1 March 1977 (1977-03-01), pages 93-98, XP000937595, ISSN: 0125-1562
- JACINTO-JAMORA S ET AL: "Pityrosporum folliculitis in the Philippines: Diagnosis, prevalence, and management", JOURNAL OF THE AMERICAN ACADEMY OF DERMATOLOGY, MOSBY, INC, US, vol. 24, no. 5, 1 May 1991 (1991-05-01), pages 693-696, XP025616620, ISSN: 0190-9622, DOI: 10.1016/0190-9622(91)70104-A [retrieved on 1991-05-01]
- LEI G -S ET AL: "All-Trans Retinoic Acid in Combination with Primaquine Clears Pneumocystis Infection", PLOS ONE 20130104 PUBLIC LIBRARY OF SCIENCE USA, vol. 8, no. 1, 4 January 2013 (2013-01-04) , XP002762413, ISSN: 1932-6203
- S Daenen ET AL: "RETINOIC ACID AS ANTILEUKEMIC THERAPY IN A PATIENT WITH ACUTE PROMYELOCYTIC LEUKEMIA AND ASPERGILLUS PNEUMONIA", Blood, 1 February 1986 (1986-02-01), pages 559-561, XP055306367, UNITED STATES Retrieved from the Internet: URL:http://www.bloodjournal.org/content/67 /2/559.full.pdf
- TILMAN E. KLASSERT ET AL: "Modulatory role of vitamin A on the Candida albicans-induced immune response in human monocytes", MEDICAL MICROBIOLOGY AND IMMUNOLOGY, vol. 203, no. 6, 17 August 2014 (2014-08-17), pages 415-424, XP55306606, DE ISSN: 0300-8584, DOI: 10.1007/s00430-014-0351-4
- ELENA CAMPIONE ET AL: "Fungistatic activity of all-trans retinoic acid against Aspergillus fumigatus and Candida albicans", DRUG DESIGN, DEVELOPMENT AND THERAPY, 1 April 2016 (2016-04-01), page 1551, XP55306124, DOI: 10.2147/DDDT.S93985

## Description

### Summary

The present invention relates to a pharmaceutical composition comprising or consisting of all-trans-retinoic acid for use in the treatment of fungal infections.

### Background of the Invention

All-transretinoic acid (ATRA) is a vitamin A derivative used in the treatment of blood cancers (promyelocytic leukemia) has antitumor action through anti-proliferative mechanisms, pro-differentiating, anti-metastatic, antiangiogenic and pro-apoptotic actions. Based on our previous experiments performed with another retinoid for the treatment of onychomycosis caused by *Candida albicans* and *Tricophyton,* we then studied and demonstrated in vitro the direct fungistatic effect of all-trans-retinoic acid in the treatment of *Candida and Aspergillus fumigatus.*

Retinoic acid has been used for the treatment of tinea versicolor (Handojo et al., Southeast Asian Journal of Tropical Medicine and Public Health, vol. 8, no.1, 1 March 1977, pages 93-98; WO 03/039418 A1; WO 97/02030 A1), infection with *Pityrosporum folliculitis* (Jacinto-Jamora et al., Journal of the Academy of Dermatology, vol. 24, no. 5, 1 May 1991, pages 693-696), Pneumocystis (Lee et al., PLOS One, vol. 8, no.1, 4 January 2013), and treatment of damage caused by reactive oxygen metabolites (WO 03/039418 A1). The use of 13-cis retinoic acid in the treatment of aspergillus infection in patients with acute promyelocytic leukemia is known from (Daenen et al; Blood, 1 February 1986, pages 559-561).

### Detailed Description of the Invention

We investigated the efficacy of ATRA at different concentrations for its antifungal activity against opportunistic *Aspergillus fumigatus* and *Candida albicans* obtained from clinical samples according to standard protocols. A fungistatic activity of ATRA at 0.5-1 mM concentration on *Aspergillus fumigatus* and *Candida albicans* was documented up to seven days.

### Figures

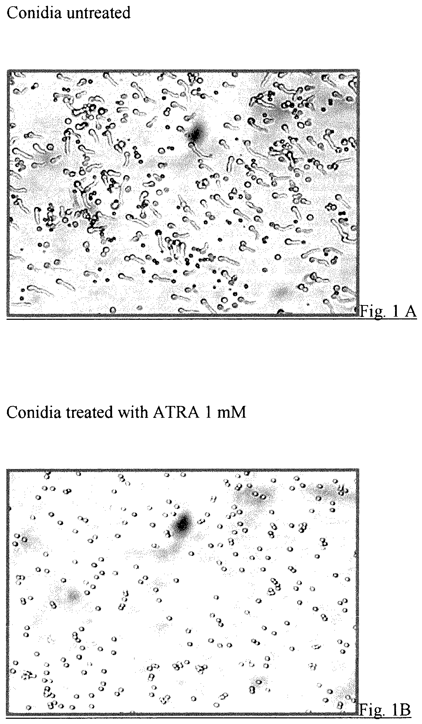

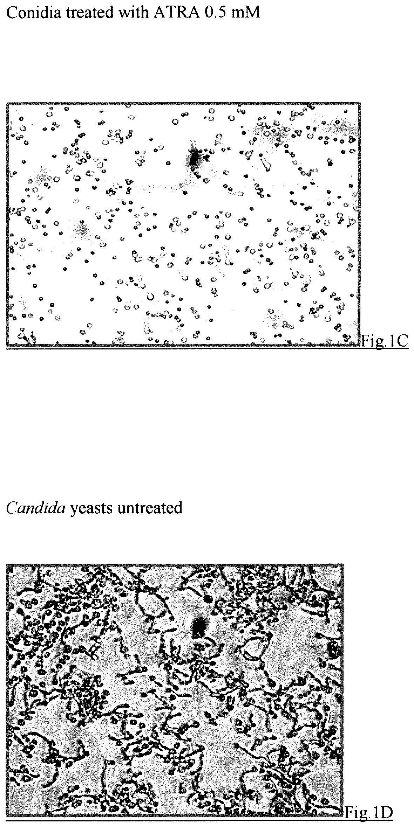

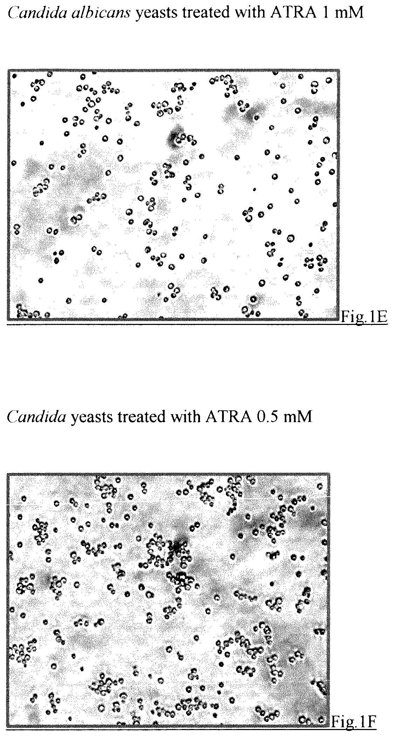

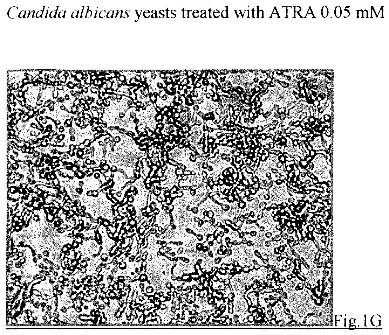

**FIG. 1****. Inhibitory effect of ATRA on *Aspergillus* and *Candida* germination.** Resting *Aspergillus fumigatus* conidia or *Candida albicans* yeast cells were culture in RPMI 1640 with 10% FCS at 37°C in the presence or absence of ATRA. **A**. Conidia untreated; **B**. Conidia treated with ATRA 1 mM; **C**. Conidia treated with ATRA 0.5 mM; **D**. *Candida* yeasts untreated; **E**. *Candida albicans* yeasts treated with ATRA 1 mM; **F**. *Candida* yeasts treated with ATRA 0.5 mM; **G**. *Candida albicans* yeasts treated with ATRA 0.05 mM. The germination of both fungi was followed using an optical microscope.

Microscopic images were recorded after 8 and 4 h of incubation for *Aspergillus fumigatus* conidia and *Candida albicans* yeasts, respectively.

### Examples

### Methods

### Fungal culture conditions

*Aspergillus fumigatus* and *Candida albicans* strains used in this study were obtained from clinical sample. The fungal strains were grown on Sabouraud dextrose agar (Difco, Detroit, MI) supplemented with chloramphenicol. Resting conidia and Candida yeasts were harvested by washing the slant cultures with sterile saline. Swollen conidia were obtained by shaking at 300 rpm at 37° C for 8 h in RPMI 1640 with 10% of fetal calf serum (FCS), until the spores swelled to almost twice their resting diameter. For generation of hyphae, swollen conidia were allowed to germinate (98% germination) by further incubation in RPMI 1640 with 10% FCS (∼ 6 h). For generation of hyphae, *Candida albicans* yeasts were allowed to germinate (98% germination) by incubation at 37°C for 4 h in RPMI 1640, PH 6.8-7.2, with 10% FCS. To assess the effect of ATRA on the germination of *Aspergillus fumigatus* or *Candida albicans*, resting conidia or yeast cells were plated at the density of 1x10⁵ in 24 well-microplate in 1 ml of RPMI 1640 with 10% FCS and supplemented with penicillin (100 U/ml) and streptomycin (100 µg/ml). ATRA (Sigma-Aldrich, Milan, Italy) was added to fungal suspension culture at time 0 at different concentrations (1, 0.5, 0.25, 0.12 and 0.06 mM). Fungal cultures were maintained at 37° C under continuous shaking at 300 rpm, and monitored every day until 1 week following treatment. Vitality of *Candida albicans* yeasts and *Aspergillus fumigatus* conidia was evaluated by light microscopy by the trypan blue dye exclusion method. To confirm vitality after exposure to higher doses of ATRA for 7 days, conidia and yeast cells were harvested, washed, and again cultured with fresh medium containing 10% FCS, in the absence of ATRA. Under this condition conidia and yeasts were able to germinate into hyphae within 24 h.

### Evaluation of fungistatic activity

*Aspergillus fumigatus* and *Candida albicans* development was followed using an optical microscope (Carl Zeiss, United Kingdom) with a 40x magnification objective lens. Microscopic images were recorded after 8 and 4 h of incubation with ATRA at 37° C for *Aspergillus fumigatus* conidial swelling and *Candida albicans* yeast cell germination, respectively.

### Results

Light optical microscopy revealed that, at earliest time point (8 h) in the presence of ATRA (1 mM), *Aspergillus fumigatus* conidia did not swell and remained similar in shape and size to resting ones (Fig. 1B). On the contrary, as shown in Fig. 1A, most of control untreated conidia (∼ 80%) were swollen and some developed short germ tube like those observed in ATRA-treated cultures of non-synchronized population of *Aspergillus fumigatus* at doses < 1mM (Fig.1C). After more prolonged incubation (24 h), all control and ATRA-treated conidia at doses < 1 mM were swollen and produced a germ tube (data not shown). ATRA at 1 and 0.5 mM also inhibited germination and hyphal outgrowth of *Candida albicans* (Fig.1E and F). Instead, control untreated yeast cells developed germ tube and subsequently hyphal growth (Fig. 1 D). Doses of ATRA < 0.5 mM did not affect *Candida albicans* ability to germinate (Fig. 1 G). The inhibitory effect of ATRA on *Aspergillus fumigatus* and *Candida albicans* germination was maintained at examined later time point (day 7). These data suggested that Fungistatic activity of ATRA at 0.5-1 mM concentration was obtained without any adverse effect on *Aspergillus fumigatus* and *Candida albicans* yeast and conidia, since their viability was > 90% as assessed by trypan blue.

The same was observed at other time points (data not shown). To confirm vitality of *Aspergillus fumigatus* and *Candida albicans* after exposure to higher (0.5-1 mM) doses, conidia and yeast cells were harvested, washed after 7 days, and again cultured in the absence of ATRA. *Aspergillus fumigatus* and *Candida albicans* conidia and yeasts were able to germinate into hyphae within 24 h (data not shown), again supporting ATRA fungistatic activity *in vitro.*

## Claims

1. A pharmaceutical composition comprising or consisting of all-trans-retinoic acid for use in the treatment of a fungal infection, wherein the fungal infection is caused by a fungal pathogen selected from the group consisting of *Candida albicans* and *Aspergillus fumigatus.*

## Patentansprüche

1. Pharmazeutische Zusammensetzung, umfassend oder bestehend aus all-trans-Retinsäure, zur Anwendung bei der Behandlung einer Pilzinfektion, wobei die Pilzinfektion durch einen Pilzpathogen verursacht wird, der aus der Gruppe bestehend aus, Candida albicans und Aspergillus fumigatus, ausgewählt ist.

## Revendications

1. Composition pharmaceutique comprenant ou consistant de l'acide tout-trans rétinoïque pour son utilisation dans le traitement d'une infection fongique, l'infection fongique étant provoquée par un pathogène fongique choisi parmi le groupe consistant en Candida albicans et Aspergillus fumigatus.
